# EUROPÄISCHE PATENTSCHRIFT

(11) **EP 1 604 018 B1**
(45) Veröffentlichungstag und Bekanntmachung des Hinweises auf die Patenterteilung: **26.09.2007**
(21) Anmeldenummer: 04721127.1
(22) Anmeldetag: 17.03.2004
(51) Int. Cl.: C12N 5/08, C12N 5/02

(54) **KNORPELZELL-KULTURMEDIUM UND VERWENDUNG DESSELBEN**
CARTILAGINOUS CELL CULTURE MEDIUM AND THE USE THEREOF
MILIEU DE CULTURE DE CELLULES CARTILAGINEUSES ET UTILISATION DE CE MILIEU DE CULTURE

(30) Priorität: 17.03.2003 DE 10311620
(43) Veröffentlichungstag der Anmeldung: 14.12.2005
(73) Patentinhaber: Biotissue Technologies GmbH, 79108 Freiburg (DE)
(72) Erfinder: ALDRIAN, Christine, 79104 Freiburg (DE)
(74) Vertreter: Schwabe - Sandmair - Marx
(86) Internationale Anmeldenummer: PCT/EP2004/002753
(87) Internationale Veröffentlichungsnummer: WO 2004/083415

(56) Entgegenhaltungen:
- WO-A-00/27996
- WO-A-98/59035
- US-A- 6 150 163
- "OptiPro(TM) SFM" [Online] Juni 2001 (2001-06), , XP002293964 Gefunden im Internet: URL:http://www.invitrogen.com/content/sfs/ manuals/3943.pdf>

## Beschreibung

Die vorliegende Erfindung betrifft ein für Knorpelzellen geeignetes und vorzugsweise für Knorpelzellen selektives Kulturmedium.

### Hintergrund der Erfindung

Die Knorpelzellen (Chondrozyten) sind Zellen, die für die Bildung von Knorpelgewebe vor allem in Gelenken verantwortlich sind. Verletzungen des Knorpels beispielsweise aufgrund von Unfällen oder altersbedingte und/oder verschleißbedingte Schäden des Knorpel führen meist zu einer erheblichen Beeinträchtigung der Beweglichkeit und zu Schmerzen beim Betroffenen.

Defekte von Knorpelgewebe ließen dem Mediziner früher nur die Wahl, das betroffene Gelenk ruhig zu stellen. Im Rahmen der modernen Transplantationsmedizin können derartige Defekte heute häufig durch Transplantation entsprechenden Gewebematerials gelindert oder behoben werden.

Üblicherweise werden die Zellen bei einem derartigen Verfahren in eine dreidimensionale Trägerstruktur eingelagert, wobei die Form der Trägerstruktur meist der Form des späteren Implantates entspricht. Die US-Patente der Nr. 5 053 050 und 5 736 372 beschreiben Zusammensetzungen zur Reparatur von Knorpel oder Knochen, wobei Knorpel- oder Knochenzellen in eine biologisch resorbierbare Trägersubstanz eingebracht werden. Zur Stützung der Zellkultur können der Nährlösung das Zellwachstum fördernde Substanzen, Adhäsionsfaktoren usw. zugesetzt werden.

Die WO 98/59035 betrifft ein serumfreies Zellkulturmedium. Es wird ein chemisch definiertes, serumfreies Kulturmedium für *in vitro* und *ex vivo* Zellen und Zelllinien offenbart. Das Medium besteht aus einem in etwa 1 : 1 Verhältnis (v/v) aus zwei basalen Kulturmedien, enthaltend α-Ketoglutarat, Insulin, Transferrin, Selen, Rinderserumalbumin, Linolsäure, Ceruloplasmin, Cholersterol, Phosphatidyl-Ethanolamin, α-Tocopherolsäuresuccinat, reduziertes Glutathion, Taurin, Triiodthyronin, Hydrocortison, Parathyroid-Hormon, L-Ascorbinsäure-2-Sulfat, β-Gylcerolphosphat, PDGF, EGF und FGF. Wenn in diesem Medium Chondrozyten in der Anwesenheit von morphogenetischen Protein abgeleitet von Knorpel oder Knochen-Morphogenitischem Protein kultiviert werden, behalten sie ihren Knorpelphenotyp.

Die WO 00/27996 betrifft ein serumfreies Medium für chondrozytenähnliche Zellen. Es wird ein Serumsersatz zur *in vitro* Kultivierung unter Verwendung definierter Faktoren, die in der Lage sind, die Lebensfähigkeit, Proliferation und Differenzierung ebenso effektiv wie ein Serum enthaltenden Medium zu unterstützen, offenbart. In einer bevorzugten Ausführungsform sind die Zellen Gelenk-Chondrozyten oder mesenchymale Stammzellen.

Des Weiteren betrifft die US 6,150,163 Zusammensetzungen für Chondrozytenmedien sowie Kulturverfahren. Eine Zielsetzung dieses Dokuments basiert auf der Entwicklungen und der Verwendung eines serumfreien definierten Zellkulturmediums, umfassend eine Ergänzungsmischung, eine Komponentenmischung, eine Vitaminmischung, eine anorganische Salzmischung und eine Aminosäuremischung, die die Probleme verhindert, die mit der Verwendung von Serum einhergehen. Insbesondere ist das definierte Medium nützlich in der Kultivierung von Fibroblasten, insbesondere Chondrozyten. Eine weitere Zielsetzung dieses Dokumentes ist es, ein Verfahren zu beanspruchen, das die Differenzierung der Chondrozyten verbessert und die Synthese einer knorpelspezifischen Matrix zur Verwendung des Tumorwachstumsfaktors beta-(TGF-β) verbessert. Das Dokument beschreibt des Weiteren ein Verfahren, das die Differenzierung der Chondrozyten unter Verwendung der Kombinationen aus TGF-β und IGF verbessert.

Gelenkknorpelersatz bzw. Knorpeltransplantate, bestehend aus Trägermaterial (bspw. einem resorbierbaren Vlies), biologischer Matrix (bspw. einem Gel) und autologen Zellen, insbesondere Chondrozyten, wird bzw. werden üblicherweise durch Anzucht der Chondrozyten aus dem Biopsat in 3-D-Kultur für ca. 7 Wochen in einem RPMI-Medium, Einbettung der Chondrozyten in die Matrix bspw. durch Einwalken sowie eine anschließende 2 - 5-tägige Vorkultivierung im Vlies erzeugt. Jedes für die Züchtung der Knorpelzellen verwendete Kulturmedium muss folgendes leisten:

Ersten muss es auf die Knorpelzellen proliferationsfördernd wirken. Zweitens sollte es die Dedifferenzierung der Chondrozyten hin zu einem Fibroblastoiden Typus möglichst hinauszögern. Da Dedifferenzierung and starke Poliferation meist eng zusammenhängen, muss ein geeigneter Kompromiß zwischen wachstumsfördernden und die Differenzierung stabilisierenden Eigenschaften gefunden werden. Schließlich muss ein Kulturmedium den chondrogenen Charakter der Knorpelzellen erhalten.

Kommerziell für medizinische Zwecke wird derzeit üblicherweise als Kulturmedium RPMI 1640 (enthaltend: Glucose, Aminosäuren, Vitamine, Salze und Carbonat-Puffer) ggf. supplementiert mit Humanserum, meist 10 - 15 %, verwendet. Letzteres muss vom Spender gewonnen werden, wobei die Gewinnung für diesen eine weitere Belastung darstellt. Die zu transplantierenden Zellen werden in diesem Medium üblicherweise ca. 7 Wochen bis zur gewünschten Zellzahl kultiviert. Nach dem Beimpfen auf den Transplantatträger müssen die Zellen bis zur Transplantation noch 2-5 Tage im Differenzierungsmedium kultiviert werden.

Für wissenschaftliche Zwecke verwendet man als Kulturmedium in der Regel ein mit FCS (meist 15 %) supplementiertes RPMI-Medium. Dies ist für die medizinische Verwendung ausgeschlossen.

Es ist daher Aufgabe der vorliegenden Erfindung, ein Knorpelzell-Kulturmedium bereitzustellen, welches die oben genannten Kriterien erfüllt. Es ist weiterhin Aufgabe der vorliegenden Erfindung, ein Knorpelzell-Kulturmedium bereitzustellen, welches eine Verkürzung der Kulturzeiten bis zur Erzielung der gewünschten Zellzahl und/oder eine Verringerung der Zeitspanne bis zum Beimpfen des Transplantatträgers gestattet. Eine Minimierung des Gehalts an Humanserum vereinfacht die Logistik und stellt eine wesentliche Erleichterung für den Patienten dar.

### Zusammenfassung der Erfindung

Die genannten Aufgaben werden erfindungsgemäß gelöst durch ein Knorpelzell-(Chondrozyten)-Kulturmedium, enthaltend ein für die Kultur von primären human Zellen geeignetes Basismedium, den zusätzlich folgende Wachstumsfaktoren zugesetzt werden: 0,5 - 50,0 ng/ml EGF, 0,5 - 50,0 ng/ml FGF, 0,1 - 10,0 ng/ml PDGF, 0,5 - 50,0 ng/ml IGF, 1,0 - 100,0 ng/ml Dexamethason and 0,1 - 10,0 mM Glutamin. Dieses kann, falls gewünscht, mit 0 - 15 % Humanserum supplementiert werden.

In einem zweiten Aspekt betrifft die Erfindung die Verwendung eines wie oben definierten Knorpelzell-Kulturmediums zur Züchtung von Knorpelzellen.

### Kurze Beschreibung der Zeichnung

Fig.1 zeigt in Form eines Balkendiagrammes die Zellzahl nach 8tägiger Kultur in verschiedenen Kulturmedien, erhalten gemäß Beispiel 1.

Fig.2 zeigt die Zeit bis zum Erhalt einer gewünschten Zellzahl am Vergleich dreier Biopsate in herkömmlichem Medium (RPMI) und einem erfindungsgemäßen Medium.

### Genaue Beschreibung der Erfindung

Wie oben beschrieben, betrifft die Erfindung ein spezielles, wie in den Ansprüchen definiertes Knorpelzell-Kulturmedium. Dieses Knorpelzell-Kulturmedium enthält vorzugsweise ausschließlich nach dem Deutschen Arzneimittelgesetz (AMG) sowie nach den good medical practice- (GMP)-Richtlinien der EU zur Verwendung am Menschen zugelassene Substanzen. Die aus der Kultur gewonnenen Knorpelzellen (Chondrozyten) können damit ohne Bedenken hinsichtlich des Kultunnediums als Transplantat verwendet werden.

Das erfindungsgemäße Knorpelzell-Kulturmedium enthält ein für die Kultur von primären human Zellen geeignetes, vorzugsweise reichhaltiges Basismedium, das zusätzlich enthält: 0,5 - 50,0 ng/ml EGF, 0,5 - 50,0 ng/ml FGF, 0,1 - 10,0 ng/inl PDGF, 0,5 - 50,0 ng/ml IGF, 1,0 - 100,0 ng/ml Dexamethason and 0,1 - 10,0 mM Glutamin. Vorzugsweise enthält das Knorpelzell-Kulturmedium zusätzlich 5,0 - 20,0 ng/ml EGF, 5,0 - 20,0 ng/ml FGF, 1,0 - 6,0 ng/ml PDGF, 5,0 - 20,0 ng/ml IGF, 10,0 - 60,0 ng/ml Dexamethason and 1,0 -6,0 mM Glutamin, noch stärker bevorzugt 10,0 ng/ml EGF, 10,0 ng/ml FGF, 3,0 ng/ml PDGF, 10,0 ng/ml IGF, 40,0 ng/ml Dexamethason und 4,0 mM Glutamin.

Erfindungsgemäß kann grundsätzlich jedes bekannte und für die Kultivierung von primären human Zellen geeignete Basismedium verwendet werden. Bevorzugt handelt es sich um ein relativ reichhaltiges Medium (kein Mangelmedium). Das Basismedium kann jede geeignete Kohlenstoff- und/oder Stickstoffquelle verwenden. Vorzugsweise werden Kohlenstoff und Stickstoffquellen verwendet, die den Vorgaben des AMG und der GMP-Richtlinie entsprechen. Die Konzentrationen sind hier variabel und können vom Fachmann anhand von Routineversuchen bestimmt und optimiert werden. Das Medium weist weiterhin vorzugsweise einen Ca²⁺-Gehalt von nicht über 2,5 mM, vorzugsweise nicht über 1,8 mM auf. Bevorzugt liegt der Gehalt an Ca²⁺ außerdem nicht unter 0,4 mM, stärker bevorzugt nicht unter 0,6 mM.

Geeignete und für die Zwecke der vorliegenden Erfindung bevorzugte Medien sind ausgewählt aus der Gruppe, bestehend aus Eagle's MEM, DMEM, MEM, RPMI und OptiPro. Ein besonders bevorzugtes Medium ist das Basalmedium OptiPro®, das von der Firma Invitrogen, vertrieben wird.

Der erste Zusatz des erfindungsgemäßen Knorpelzell-Kulturmediums ist ein EFG (Epidermal Growth Factor), in einer Menge von 0,5 bis 50,0 ng/ml, vorzugsweise 5,0 bis 20,0 ng/ml, am meisten bevorzugt 10,0 ng/ml. Der EGF ist ein genereller Wachstumsfaktor für mesenchymale Zellen. Bevorzugt handelt es sich um rekombinantes, noch stärker bevorzugt rekombinantes human EGF (rh EGF).

Der zweite Zusatz des erfindungsgemäßen Knorpelzell-Kulturmediums ist ein FGF, vorzugsweise der basic FGF (Fibroblast Growth Factor), in einer Menge von 0,5 bis 50,0 ng,/ml, vorzugsweise 5,0 bis 20,0 ng/ml, am meisten bevorzugt 10,0 ng/ml. Der bFGF ist in vivo ein genereller Wachstumsfaktor für mesodermale und neuro-ectodermale Zellen. In vitro schränkt der bFGF die Ansprüche an die Serumqualität ein. Bevorzugt handelt es sich um einen rekombinanten, noch starker bevorzugt einen rekombinanten, humanen bFGF.

Der dritte Zusatz des erfindungsgemäßen Knorpelzell-Kulturmediums ist der PDGF (Plathelet Derived Growth Factor) in einer Menge von 0,1 bis 10,0 ng/ml, vorzugsweise 1,0 bis 6,0 ng/ml, am meisten bevorzugt 3,0 ng/ml. Unter den Oberbegriff PDGF fällt ein ganzes Spektrum an verwandten Molekülen, denen verschiedene Aufgaben zukommen. U.a. fungieren die PDGF als Wachstumsfaktoren, bei der Wundheilung und als Chemotaxisfaktoren. Bevorzugt verwendet man einen rekombinanten human PDGF (rh PDGF). Stärker bevorzugt handelt es sich um BB-Homodimere.

Der vierte Zusatz des erfindungsgemäßen Knorpelzell-Kulturmediums ist ein IGF, vorzugsweise IGF-1 (Insulinlike Growth Factor), in einer Menge von 0,5 bis 50,0 ng/ml, vorzugsweise 5,0 bis 20,0 ng/ml, am meisten bevorzugt 10,0 ng/ml. Der Ausdruck IGF bezeichnet als Oberbegriff insbesondere den IGF-1 und den IGF-2. Die IGF stellen starke Wachstumsfaktoren u.a. für Knorpelzellen dar. Sie wirken zum Teil synergistisch mit anderen Wachstumsfaktoren. Bevorzugt verwendet man einen rekombinanten, stärker bevorzugt rekombinanten human IGF (rh IGF).

Bei dem fünften Zusatz des erfindungsgemäßen Knorpelzell-Kulturmediums handelt es sich um Dexamethason in einer Menge von 1,0 bis 100,0 ng/ml, vorzugsweise 10,0 bis 60,0 ng/ml, am meisten bevorzugt 40,0 ng/ml. Dexamethason (9a-Fluor-16a-methylprednisolon) ist ein halbsynthetisches Glucocorticoid, welches die ACTH-Sekretion beeinflusst. Dexamethason ist ein Differenzierungsfaktor für Knorpelzellen.

Bei dem sechsten Zusatz des erfindungsgemäßen Knorpelzell-Kulturmediums handelt es sich um Glutamin in einer Menge von 0,1 bis 10,0 mM, vorzugsweise 1,0 bis 6,0 mM, am meisten bevorzugt etwa 4,0 mM. Glutamin ist eine der natürlich vorkommenden Aminosäuren, die üblicherweise als Stickstoffquelle dienen kann.

Weiterhin kann, das erfindungsgemäße Medium mit Humanserum (HS), vorzugsweise autologisch, in einer Menge von 0-15 %, vorzugsweise 0-10 %, am meisten bevorzugt 3-5 % (Gew/Vol) supplementiert sein, muss aber nicht. Das zeigt, dass das erfindungsgemäße Kulturmedium eine vorteilhafte Verringerung dieses Zusatzes ermöglicht, der nach dem Stand der Technik bislang in höheren Mengen erforderlich war.

In einem zweiten Aspekt betrifft die vorliegende Erfindung die Verwendung des oben beschriebenen Knorpelzell-Kulturmediums zur Züchtung von Knorpelzellen, insbesondere zu deren selektiver Züchtung. Die Kulturbedingungen sind dem Fachmann bekannt und können anhand von Routineexperimenten optimiert werden.

Die Züchtung der Knorpelzellen unter Verwendung des erfindungsgemäßen Mediums erfolgt ausgehend von Gewebebiopsien eines Spenders und vorzugsweise des zu behandelnden Patienten selbst. Vorzugsweise sind die erhaltenen Knorpelzellen zur Reimplantation in den Menschen, insbesondere den Spender bestimmt. Sind Spender und Empfänger identisch, ist eine Transplantatabstoßung nicht zu befürchten, sofern nicht das Kulturmedium antigene Substanzen enthält. Letzteres entspricht nicht dem AMG und ist daher nicht erwünscht.

Ein überraschender und nicht zu erwartender Vorteil des erfindungsgemäßen Mediums mit der erfindungsgemäßen Zusatzkombination sind die hohen Vermehrungsraten der Knorpelzellen, im Beispiel mit nur 3 % humanem autologen Serum. In entsprechenden Vergleichsversuchen mit dem konunerziell verwendeten Medium RPMI, supplementiert mit Humanserum (10 % und 15 %), hat die Anmelderin gefunden, dass das erfindungsgemäße Knorpelzell-Kulturmedium hinsichtlich der Wachstumsraten verbesserte Ergebnisse liefert. Konkret lässt sich die Zeit bis zum Beimpfen des Transplantatträgers und anschließend bis zur Transplantation mit Hilfe des erfindungsgemäßen Kulturmediums deutlich senken. Durch kürzere Kultivierung und optimierte Faktoren sind die Zellen weniger dedifferenziert. Das chondrogene Potential und damit die Produktqualität ist somit erhöht. Gemäß der bevorzugten Ausführungsform kann beispielsweise diese Zeit um das 3,5-fache verkürzt werden.

### Beispiele

### Beispiel 1

In einem ersten Beispiel wurden je 0,1 Mio. Zellen eines Biopsats, d.h. identischen Ursprungs, in fünf verschiedenen Medien kultiviert und die Zellzahl nach 8 Tagen in Kultur bestimmt. Die Kultur erfolgte bei 37°C in Zellkulturflaschen. Verwendet wurde als Vergleich RPMI, supplementiert mit 15 % Humanserum (HS) als das bisherige Knorpelmedium sowie das Medium OptiPro der Fa. Invitrogen und Wachstumszusätze, supplementiert mit 3 % HS:

**Tabelle 1: Verwendete Wachstumszusätze (BTT Faktormix)**

| **Zusatz** | **Menge** |
|---|---|
| EGF: | 10 ng/ml |
| FGF: | 10 ng/ml |
| PDGF: | 3 ng/ml |
| IGF: | 10 ng/ml |
| Dexamethason: | 40 ng/ml |
| Glutamin | 4 mM |

**Tabelle 2: Verwendete Medien**

| Basismedium | Humanserum (%) | BTT Faktormix |
|---|---|---|
| RPMI | 3 | + |
| RPMI (Vergl.) | 15 | - |
| OptiPro® | 3 | + |
| OptiPro© | - | + |
| OptiPro® (Vergl.) | 3 | - |

Zum direkten Vergleich wurden parallele Zellkulturen aus Biopsaten derselben Testpersonen mit den in Tabelle 2 genannten Medien etabliert und nach gängigen Verfahren geführt. Im Ergebnis entspricht das Wachstum und die Morphologie von etablierten Knorpelzell-Kulturen mit dem erfindungsgemäßen Medium etwa denjenigen des supplementierten RPMI-Mediums.

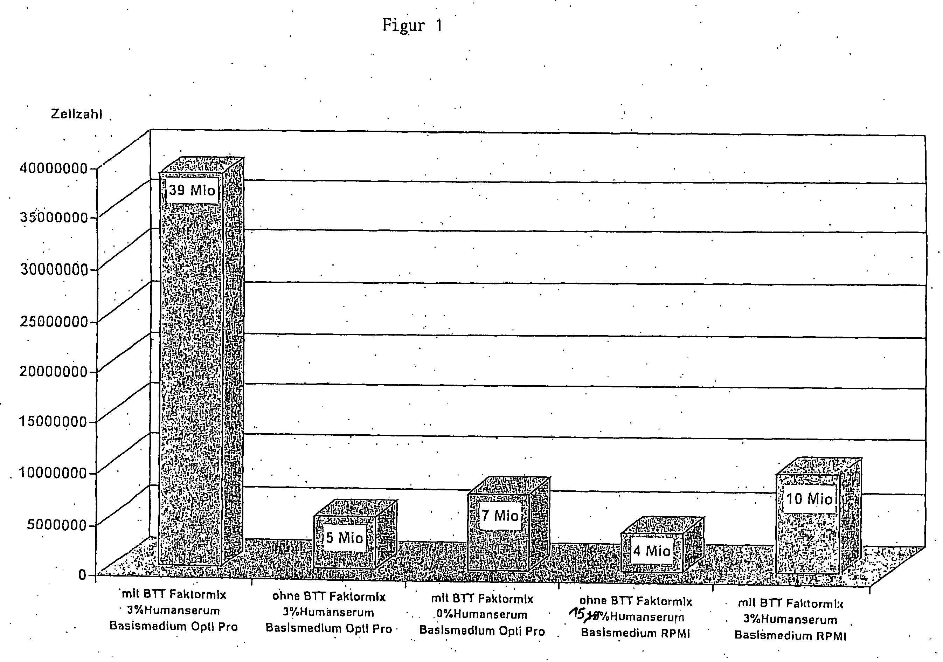

Allerdings bietet das erfindungsgemäße Medium Vorteile bei der Etablierung der Kultur, dahingehend, dass die Zellausbeute bei geringerer Supplementierung mit HS durchschnittlich 2,5 bis 10-mal höher liegt. Die Ergebnisse sind in Fig. 1 gezeigt.

Gleichzeitig lässt sich dadurch die Kulturdauer und Zahl der erforderlichen Passagierungen bis zum Erhalt der zum Beimpfen des Transplantats erforderlichen Zellzahl senken. Insgesamt kann damit die Transplantation früher und mit besserer Zellqualität erfolgen.

### Beispiel 2:

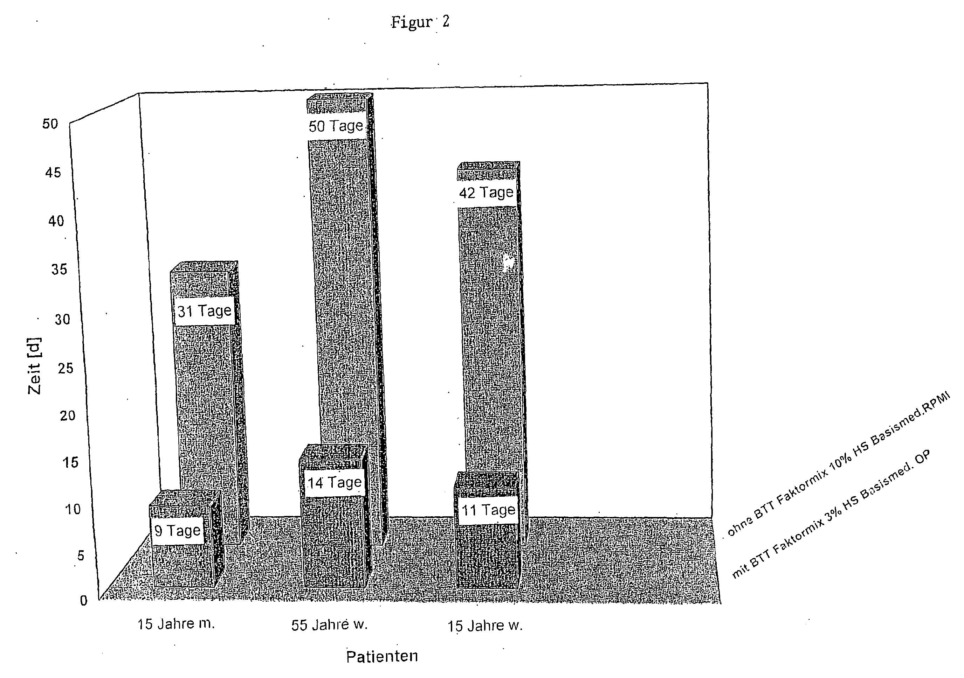

In einem zweiten Versuch wurden Biopsate dreier Spender jeweils in erfindungsgemäßem Medium (Nr.3 oben; OptiPro® mit 3% HS und Zusätzen) bzw. RPMI (10% HS) kultiviert und die Dauer bis zum Erhalt der erforderlichen Zellzahl in der Kultur, ausgehend von identischen Okulaten, bestimmt. Die Ergebnisse sind in Fig. 2 gezeigt.

Aus Fig. 2 ist ersichtlich, dass sich durch Kultivieren im erfindungsgemäßen Medium die Zeit bis zum Erhalt einer Zellzahl von 60 Mio. Zellen in Abhängigkeit vom Alter des Spenders deutlich (ca. um das 3-fache) verkürzen lässt.

## Patentansprüche

1. Knorpelzell-Kulturmedium, enthaltend ein für die Kultur von primären human Zellen geeignetes Basismedium und zusätzlich: 0,5 - 50,0 ng/ml EGF, 0,5 - 50,0 ng/ml FGF, 0,1 -10,0 ng/ml PDGF, 0,5 - 50,0 ng/ml IGF, 1,0 -100,0 ng/ml Dexamethason und 0,1 - 10,0 mM Glutamin.

2. Knorpelzell-Kulturmedium nach Anspruch 1, enthaltend: 5,0 - 20,0 ng/ml EGF, 5,0 - 20,0 ng/ml FGF, 1,0 - 6,0 ng/ml PDGF, 5,0 - 20,0 ng/ml IGF, 10,0 - 60,0 ng/ml Dexamethason und 1,0 - 6,0 mM Glutamin.

3. Knorpelzell-Kulturmedium nach Anspruch 1, enthaltend: 10,0 ng/ml EGF, 10,0 ng/ml FGF, 3,0 ng/ml PDGF, 10,0 ng/ml IGF, 40,0 ng/ml Dexamethason und 4,0 mM Glutamin.

4. Knorpelzell-Kulturmedium nach einem der vorstehenden Ansprüche, zusätzlich enthaltend bis zu 15 % (w/v) Humanserum.

5. Knorpelzell-Kulturmedium nach Anspruch 4, zusätzlich enthaltend 3 bis 5 % (w/v) Humanserum.

6. Knorpelzell-Kulturmedium nach einem der vorstehenden Ansprüche, in dem das Basismedium ausgewählt ist aus der Gruppe, bestehend aus Eagle's MEM, DMEM, RPMI, MEM und OptiPro®.

7. Verwendung eines Knorpelzell-Kulturmediums gemäß einem der vorstehenden Ansprüche zur Züchtung von Knorpelzellen.

8. Verwendung nach Anspruch 7, worin die Züchtung ausgehend von Gewebebiopsien eines Patienten erfolgt.

9. Verwendung nach Anspruch 7 oder 8, worin die Knorpelzellen zur Reimplantation in den Menschen bestimmt sind.

10. Knorpelzellsuspension in einem Medium gemäß einem der Ansprüche 1 bis 6.

## Claims

1. A cartilage cell culture medium comprising a basic medium suitable for culturing primary human cells and, additionally, 0.5 to 50.0 ng/ml of EGF, 0.5 to 50.0 ng/ml of FGF, 0.1 to 10.0 ng/ml of PDGF, 0.5 to 50.0 ng/ml of IGF, 1.0 to 100.0 ng/ml of dexamethasone, and 0.1 to 10.0 mM of glutamine.

2. A cartilage cell culture medium according to claim 1 comprising 5.0 to 20.0 ng/ml of EGF, 5.0 to 20.0 ng/ml of FGF, 1.0 to 6.0 ng/ml of PDGF, 5.0 to 20.0 ng/ml of IGF, 10.0 to 60.0 ng/ml of dexamethasone, and 1.0 to 6.0 mM of glutamine.

3. A cartilage cell culture medium according to claim 1 comprising 10.0 ng/ml of EGF, 10.0 ng/ml of FGF, 3.0 ng/ml of PDGF, 10.0 ng/ml of IGF, 40.0 ng/ml of dexamethasone, and 4.0 mM of glutamine.

4. A cartilage cell culture medium according to any of the previous claims additionally comprising up to 15 % (w/v) of human serum.

5. A cartilage cell culture medium according to claim 4 additionally comprising 3 to 5 % (w/v) of human serum.

6. A cartilage cell culture medium according to any of the previous claims wherein the basic medium is selected from the group consisting of Eagle's MEM, DMEM, RPMI, MEM and OptiPro®.

7. The use of a cartilage cell culture medium according to any of the previous claims for culturing cartilage cells.

8. The use according to claim 7, wherein said culture is started on the basis of tissue biopsies of a patient.

9. The use according to claim 7 or 8, wherein the cartilage cells are determined for reimplantation into the human patient.

10. A cartilage cell suspension in a medium according to any of the claims 1 to 6.

## Revendications

1. Milieu de culture de cellules cartilagineuses contenant un milieu de base approprié pour la culture de cellules humaines primaires et en plus: 0,5 - 50,0 ng/ml d'EGF, 0,5 - 50,0 ng/ml de FGF, 0,1 - 10,0 ng/ml de PDGF, 0,5 - 50,0 ng/ml d'IGF, 1,0 - 100,0 ng/ml de dexaméthasone et 0,1 - 10,0 mM de glutamine.

2. Milieu de culture de cellules cartilagineuses suivant la revendication 1, contenant: 5,0 - 20,0 ng/ml d'EGF, 5, 0 - 20,0 ng/ml de FGF, 1, 0 - 6,0 ng/ml de PDGF, 5,0 - 20,0 ng/ml d'IGF, _{10,0} - 60,0 ng/ml de dexaméthasone et 1,0 - 6,0 mM de glutamine.

3. Milieu de culture de cellules cartilagineuses suivant la revendication 1, contenant: 10,0 ng/ml d'EGF, 10,0 ng/ml de FGF, 3,0 ng/ml de PDGF, 10,0 ng/ml d'IGF, 40,0 ng/ml de dexaméthasone et 4,0 mM de glutamine.

4. Milieu de culture de cellules cartilagineuses suivant l'une des revendications précédentes, contenant en outre jusqu'à 15 % (w/v) de sérum humain.

5. Milieu de culture de cellules cartilagineuses suivant la revendication 4, contenant en outre 3 à 5 % (w/v) de sérum humain.

6. Milieu de culture de cellules cartilagineuses suivant l'une des revendications précédentes, dans lequel le milieu de base est sélectionné parmi le groupe constitué de Eagle's MEM, DMEM, RPMI, MEM et OptiPro®.

7. Utilisation d'un milieu de culture de cellules cartilagineuses suivant l'une des revendications précédentes pour la culture de cellules cartilagineuses.

8. Utilisation suivant la revendication 7, pour laquelle la culture est basée sur des biopsies tissulaires d'un patient.

9. Utilisation suivant la revendication 7 ou 8, pour laquelle les cellules cartilagineuses sont destinées à la réimplantation dans l'être humain.

10. Suspension de cellules cartilagineuses dans un milieu suivant l'une des revendications 1 à 6.
